# EUROPÄISCHE PATENTANMELDUNG

(11) **EP 1 321 156 A1**
(43) Veröffentlichungstag der Anmeldung: **25.06.2003**
(21) Anmeldenummer: 01130138.9
(22) Anmeldetag: 19.12.2001
(51) Int. Cl.: A61M 5/168, G05D 16/06

(54) **Ventil zum weitgehenden Konstanthalten eines Flüssigkeitsstromes**

(71) Anmelder: Wex, Roland, 34212 Melsungen (DE)
(72) Erfinder: Wex, Roland, 34212 Melsungen (DE); Hansmann, Harald, Dr., 53797 Lohmar (DE)
(74) Vertreter: Quermann, Helmut, Dipl.-Ing.

(57) **Zusammenfassung**

Die Erfindung betrifft ein Ventil (1), insbesondere für medizinische und ernährungsphysiologische Flüssigkeiten, sowie Flüssigkeiten im biologischen und Laborbereich, mit einem Gehäuse (2), das eine Kammer (6), einen in Strömungsverbindung mit der Kammer stehenden Eintrittskanal (5) und einen in Strömungsverbindung mit der Kammer stehenden Austrittskanal (7) für die Flüssigkeit aufweist, wobei innerhalb der Kammer ein Ventilkörper (15) zum Regeln des Durchflusses der Flüssigkeit durch die Kammer angeordnet ist.

Gemäß der Erfindung wird vorgeschlagen, dass der Ventilkörper beweglich ist, sowie mit einem elastischen Element (13) verbunden ist, das Bestandteil der Kammerwand (16) bildet.

Ein solches Ventil ermöglicht ein weitgehendes Konstanthalten der Flowrate.

## Beschreibung

Die Erfindung betrifft ein Ventil, insbesondere für medizinische und ernährungsphysiologische Flüssigkeiten, sowie Flüssigkeiten im biologischen und Laborbereich, mit einem Gehäuse, das eine Kammer, einen in Strömungsverbindung mit der Kammer stehenden Flüssigkeitskanal und einen in Strömungsverbindung mit der Kammer stehenden Austrittskanal für die Flüssigkeit aufweist, wobei innerhalb der Kammer ein Ventilkörper zum Regeln des Durchflusses der Flüssigkeit durch die Kammer angeordnet ist.

Im Zusammenhang mit der Förderung medizinischer, ernährungsphysiologischer oder biologischer Flüssigkeiten oder Flüssigkeiten im Laborbereich werden die unterschiedlichsten Typen von Flüssigkeitspumpen verwendet. Es sind beispielsweise elektroenergetisch, elektrochemisch, gasförmig, mechanisch, elektromechanisch und mechanischphysikalisch betriebene Pumpen bekannt. Viele dieser Pumpen können in aller Regel nur nach einer längeren Anlaufzeit eingesetzt werden und bieten dem Anwender und Verbraucher oftmals nur eine unzureichende Dosiergenauigkeit. Die bekannten Pumpensysteme sind im Übrigen recht kostenaufwendig und unter ökologischem Gesichtspunkt wenig vorteilhaft.

Nicht elektrisch betriebene Pumpen, vorzugsweise mechanische Infusionspumpen, haben den Nachteil, dass sie innerhalb der Einsatzzeit unterschiedliche Drücke erzeugen. D. h. der Anfangsdruck ist in der Regel höher als der Enddruck. Die Flowratenregelung wird über Kapillarschläuche oder Glaskapillarrohre vorgenommen. Diese Kapillarlösungen ergeben innerhalb der Anwendungszeit Flowratenschwankungen von bis zu 70%. Dies ist für Patienten mit Toleranzproblemen, z. B. Allergikern ein großes Problem, da zu hohe Dosierraten zu Nebenwirkungen und Unverträglichkeiten führen, bis hin zur Todesfolge.

Aus der WO/0078377 A1 ist eine mechanisch betriebene Flüssigkeitspumpe für medizinische und ernährungsphysiologische Flüssigkeiten bekannt, bei der ein Ventil zum weitgehenden Konstanthalten des von der Pumpe ausgegebenen Volumenstroms Verwendung findet. Das Ventil weist ein Gehäuse und einen von diesem aufgenommenen Ventilkörper, der mit einem Flüssigkeitskanal versehen ist, auf, wobei der Ventilkörper als elastischer Schließkörper mit äußerer Druckfläche ausgebildet ist und auf die Druckfläche ausgebildet ist und auf die Druckfläche der stromaufwärts des Ventilkörpers wirksame Druck mit einer Druckkraftkomponente senkrecht zur Längsrichtung des Flüssigkeitskanals, auf diesen hingerichtet, einwirkt.

Ein Ventil der eingangs genannten Art ist aus der EP 0 474 069 A1 bekannt. Dort ist ein plattenförmiger, elastischer Ventilkörper in seinem Randbereich eingespannt und es tritt die Flüssigkeit durch eine sich erweiternde zentrale Öffnung des Ventilkörpers aus der Kammer in den Austrittskanal aus. In Abhängigkeit von dem Anstieg des Flüssigkeitsdrucks in der Kammer vergrößert sich die Öffnung im Ventilkörper. Das Ventil weist zusätzlich die Funktion eines Rückschlagventils auf.

In der US 4 278 083 ist ein Ventil beschrieben, durch das zwei Flüssigkeitsströme geführt werden. Ein erster kontinuierlicher Flüssigkeitsstrom medizinischer Flüssigkeit durchströmt den Flüssigkeitskanal im nichtelastischen Ventilkörper, während ein zweiter, vorübergehender, manuell gesteuerter größerer Flüssigkeitsstrom am Ventilkörper vorbei zu einem Kathetersystem gelangt. Aufgrund des Umstandes, dass ein elastisches Element den Ventilkörper umschließt, kann durch Drücken des elastischen Elementes erreicht werden, dass ein Spalt zwischen diesem und dem Ventilkörper gebildet ist, der das Vorbeiströmen des größeren Volumenstroms am Ventilkörper gestattet. Der Durchmesser des Flüssigkeitskanals beträgt nur einige 1/100 mm. Der Flüssigkeitskanal stellt einen Kapillarabschnitt des Ventilkörpers dar und lässt demzufolge nur wenig Flüssigkeit pro Zeiteinheit durch.

Aus der CH 672 850 ist ein Ventil bekannt, bei dem das Fluid in zwei Teilströme aufgeteilt wird, wobei ein erster, mit ständig offener Durchflussöffnung und bei steigendem Wirkdruck anwachsender Teilstrom größer ist als ein zweiter, in gleichem Maße abfallender Teilstrom. Dieser durchströmt einen Querschnitt des Ventils, der durch ein vom Wirkdruck abhängiges elastomeres Drosselstück geregelt wird.

Weitere Ventile, die im Bereich der Medizintechnik bzw. des Gesundheitswesens Verwendung finden, sind aus der US 708 166 und US 4 919 167 bekannt.

Es ist Aufgabe der vorliegenden Erfindung, ein Ventil der eingangs genannten Art so weiterzubilden, dass ein weitgehendes Konstanthalten des Flüssigkeitsstromes, somit ein Konstanthalten des Flüssigkeitsstromes in einem engen Toleranzbereich, gewährleistet ist.
Gelöst wird die Aufgabe bei einem Ventil der eingangs genannten Art dadurch, dass der Ventilkörper beweglich sowie mit einem elastischen Element verbunden ist, das Bestandteil der Kammerwand bildet.

Erfindungsgemäß ist damit vorgesehen, dass der Ventilkörper die Funktion eines aktiven Regelungselementes besitzt, dessen Position von dem in der Kammer wirkenden Flüssigkeitsdruck abhängt. Steigt der Druck in der Flüssigkeitskammer, führt dies zu einer Stellbewegung des Ventilkörpers, die den Austritt der Flüssigkeit aus dem Eintrittskanal drosselt, während eine Abnahme des Flüssigkeitsdruckes in der Kammer dazu führt, dass der Ventilkörper eine Bewegung vollführt, die die Drosselung der aus dem Eintrittskanal austretenden Flüssigkeit reduziert. Einer eventuellen Schwankung des Flüssigkeitsdrucks in der Kammer wirkt eine entsprechende Bewegung des elastischen Elements und in der Konsequenz eine entsprechende Bewegung des mit diesem verbundenen Ventilkörpers entgegen. Es ist durchaus denkbar - beispielsweise in Folge eines Verschlusses des Austrittskanals -, dass bei recht hohem, in der Kammer wirkenden Flüssigkeitsdruck der Ventilkörper in eine Position überführt wird, in der keine Flüssigkeit durch den Eintrittskanal strömt. Hat sich der Druck in der Kammer durch die, durch den Austrittskanal austretende Flüssigkeit wieder reduziert, führt dies zu einer Veränderung der Position des elastischen Elementes und des Ventilkörpers, so dass der Flüssigkeitszugang zur Kammer wieder geregelt freigegeben wird.

Es wird als sehr vorteilhaft angesehen, wenn ein Durchflussbegrenzer zum Begrenzen des durch den Austrittskanal tretenden Volumenstroms vorgesehen ist. Der Durchflussbegrenzer ist beispielsweise dadurch in der Lage, den Volumenstrom, der durch den Austrittskanal tritt, zu begrenzen, weil er einen mit dem Austrittskanal in Verbindung stehenden Flüssigkeitskanal mit kleinerem Querschnitt aufweist. Durch die Wahl verschiedener Durchflussbegrenzer ist die Einstellung verschiedener konstanter Flowraten möglich, solange der eingangs des Eintrittskanals anliegende Druck einen bestimmten Wert nicht unterschreitet. Grundsätzlich ist vorgesehen, dass der Durchflussquerschnitt des Eintrittskanals größer ist als der Durchflussquerschnitt des Austrittskanals.

Gemäß einer besonderen Ausführungsform der Erfindung ist vorgesehen, dass das Gehäuse des Ventils ein Ventilsitzelement aufnimmt, das einen Kanal durchsetzt, der in Strömungsverbindung mit dem Eintrittskanal steht. In diesem Fall weist nicht das Gehäuse den komplementären Drossel- bzw. Dichtbereich für den Ventilkörper auf, sondem das Ventilsitzelement. Das Gehäuse kann somit weitgehend unabhängig gestaltet werden, ohne auf die spezifischen Gegebenheiten im Drossel- bzw. Verschließbereich Rücksicht nehmen zu müssen. Insofern kann das Gehäuse als einfaches Spritzgussteil gestaltet werden, insbesondere mehrteilig. Dies erleichtert auch die Montage des Ventilkörpers innerhalb der Kammer.

Vorteilhaft ist das Ventilsitzelement als elastischer Körper ausgebildet. Diese Gestaltung ermöglicht eine sichere Abdichtung des Eintrittskanals, wenn der Ventilkörper im Bereich des Kanals des Ventilsitzelementes an diesem anliegt. Andererseits kann die Elastizität des Ventilsitzelementes genutzt werden, um zur Rückstellung des Ventilkörpers beizutragen. Unter diesem Aspekt ist der Ventilkörper oberhalb des Ventilsitzelementes elastisch positioniert. Eine bevorzugte Ausführungsform der Erfindung sieht vor, den Ventilkörper mit mindestens einem sich am elastischen Ventilsitzelement abstützenden Distanzansatz zu versehen.

Unter dem Aspekt der Herstellung und der Montage sowie der Funktionalität, insbesondere einer präzisen Verschiebbarkeit, ist vorgesehen, dass der Ventilkörper im Wesentlichen topfförmig ausgebildet ist, mit mehreren, insbesondere drei von einer Grundplatte ausgehenden Wandungsstegen, die im Bereich ihrer freien Enden fest mit dem elastischen Element verbunden sind. Bei dieser Lagerung des Ventilkörpers ist eine neue separate Führung des Ventilkörpers im Gehäuse notwendig. Die Lagerung des Ventilkörpers erfolgt quasi schwimmend.

Eine bevorzugte Ausführungsform der Erfindung sieht vor, dass das elastische Element als Membran ausgebildet ist. Mit diesem ist der Ventilkörper fest verbunden, beispielsweise verklebt. Auf einfache Art und Weise kann die elastische Membran gelagert werden, wenn sie im Bereich Ihres Randes im Gehäuse abgedichtet gehalten ist.

Die Erfindung schlägt somit ein hydraulisches Ventil zur Regelung des Durchflusses einer Flüssigkeit vor. Das erfindungsgemäße Ventil zum Regulieren der Durchflussrate, gewährleistet Druckveränderungen oder Druckschwankungen, wie sie insbesondere bei mechanischen Pumpenantrieben gegeben sind, auszugleichen. Die vorgesehene Konstruktion besteht aus einfachen Einzelteilen, die unkompliziert zu montieren sind. Die verwendeten Materialien - Kunststoffe und elastische Materialien - lassen sich für alle chemischen und biologischen Ansprüche modifizieren. Die verwendeten Materialien besitzen eine gute chemische Beständigkeit, sind sterilisierbar und entsprechen als einmal verwendbare Artikel den Anwendungsanforderungen. Die Gehäuseanschlüsse können in allen gewünschten Anschlussvarianten ausgeführt werden, insbesondere sind sie als Luer-Lock-Anschluss ausgeführt. Die Gehäusekonstruktion erlaubt die Integration eines Vorfilters vor das Ventil. Ebenso ist es denkbar, dass im Zu- oder Ablaufbereich mehrere Eingangs- oder Ausgangskanäle angebracht werden.

Das erfindungsgemäße Ventil hält den Flüssigkeitsstrom bei unterschiedlichen Drücken auf einem vorgegebenen Sollwert konstant. Es gewährleistet eine Förderratengenauigkeit von maximal plus/minus 5 %, wie sie sonst nur von elektrisch betriebenen Pumpen erreicht wird. Das Ventil ist für Förderraten von 0,5 bis 1000 ml/h und für unterschiedliche Viskositäten geeignet. Als bevorzugtes Anwendungsgebiet wird ein Flowratenbereich von 0,5 bis 10 ml/h angesehen. Durch die Wahl verschiedener Durchflussbegrenzer lassen sich dazu konstant Innendrücke in der Kammer erzielen, die vorzugsweise den Bereich von 0,05 bis 0,6 bar abdecken. Zielgruppe des erfindungsgemäßen Ventils ist insbesondere der medizinische, ambulante Bereich, ferner die Verwendung in Notarztwagen, Lazaretten, bei Noteinsätzen anlässlich Katastrophen und dergleichen.

Weitere Merkmale der Erfindung sind in der Beschreibung der Figuren dargestellt, wobei bemerkt wird, dass alle Einzelmerkmale und alle Kombinationen von Einzelmerkmalen erfindungswesentlich sind.

In den Figuren ist die Erfindung anhand einer Ausführungsform beispielsweise dargestellt, ohne hierauf beschränkt zu sein. Es zeigt:
- Figur 1: eine räumliche Ansicht des Ventils,
- Figur 2: einen Längsmittelschnitt durch das Ventil (gemäß der Linie II-II in Figur 3),
- Figur 3: einen Querschnitt durch das Ventil (gemäß der Linie III-III in Figur 2),
- Figur 4: eine Funktionsdarstellung des durchflossenen Ventils und
- Figur 5: eine Funktionsdarstellung des geschlossenen Ventils.

Das Ventil 1 weist ein Gehäuse 2 auf, das auf den einander abgewandten Seiten mit Anschlussstutzen 3 und 4 versehen ist, die beispielsweise als Luer-Lock-Anschlüsse ausgebildet sind. Ausgehend vom Anschlussstutzen 3 ist ein Eintrittskanal 5 zu einer im Gehäuse 2 befindlichen Kammer 6 und ausgehend vom Anschlussstutzen 4 ein Austrittskanal 7 zur Kammer 6 geführt. Der Eintrittskanal weist einen Durchmesser, somit Durchflussquerschnitt auf, der größer ist als der Durchmesser des Austrittskanals 7. In den Anschlussstutzen 4 ist ein Durchflussbegrenzer 8 eingesetzt, der einen Einsatz 9 mit Flüssigkeitskanal 10 besitzt. Der Flüssigkeitskanal 10 schließt sich an den Austrittskanal 7 an, wobei der Durchmesser des Flüssigkeitskanals 10 geringer ist als der Durchmesser des Austrittskanals 7. Durch die Wahl verschiedener Durchflussbegrenzer 8, somit Ersetzen eines Durchflussbegrenzers durch einen anderen Durchflussbegrenzer können verschiedene, konstante Flowraten des Ventils eingestellt werden.

Das Gehäuse 2 umschließt die Kammer 6 nur seitlich. Auf den einander abgewandten Seiten des Gehäuses 2 sind Deckel 11 und 12 mit diesem verbunden, wobei der Deckel 11 die weitere Begrenzung der Kammer 6 darstellt. Zwischen dem Deckel 12 und dem Gehäuse 2 ist eine elastische Membran 13 angeordnet. Dieses flächige Funktionselement weist im Wesentlichen Kreisform auf, wobei es im Bereich des umlaufenden Randes zwischen dem Deckel 12 und dem Gehäuse 2 dichtend geklemmt ist. Der Deckel 12 dient infolgedessen dem Halten und Abdecken der elastischen Membran 13, so dass diese einerseits sich frei senkrecht zur Membranebene bewegen kann, andererseits sie vor Beschädigung berührgeschützt ist. Ein Ventilkörper 15, dessen Aufbau und Funktion nachfolgend näher beschrieben wird, ist unmittelbar mit der elastischen Membran 13 verbunden, beispielsweise mit dieser verklebt.

Der innerhalb der Kammer 6 angeordnete Ventilkörper 15 dient dem Regeln des Durchflusses der Flüssigkeit durch die Kammer 6. Der Ventilkörper ist beweglich und folgt der Bewegung der elastischen Membran, die Bestandteil der Kammerwand 16 bildet.

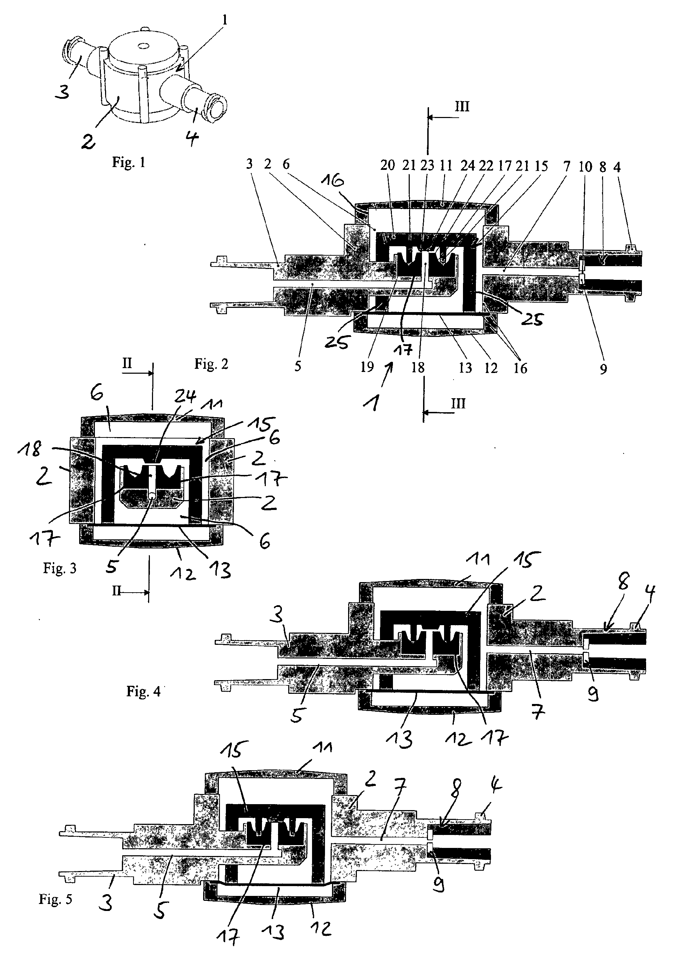

Ein in die Kammer 6 ragender Bereich des Gehäuses 2 ist mit einem Rücksprung zur Aufnahme eines rotationssymmetrischen Ventilsitzelementes 17 versehen, das einen zentralen Flüssigkeitskanal 18 aufweist. Der Flüssigkeitskanal 18, dessen Durchmesser dem Durchmesser des Eintrittskanals 5 entspricht, steht in Strömungsverbindung mit einem abgeknickten Nebenabschnitt des Eintrittskanals 5. Das Ventilsitzelement 17 besteht aus elastischem Material und ist auf der der Kammer 6 zugewandten Seite mit einer rinnenförmigen Vertiefung 19 versehen. Der Ventilkörper 15 weist eine Grundplatte 20 auf, von der sich mehrere, beispielsweise drei Distanzansätze 21 in Richtung des Ventilsitzelementes 17 erstrecken und den Boden der Vertiefung 19 des Ventilsitzelementes 17 kontaktieren. Von der Vertiefung 19 ist eine Dichtfläche 22 des Ventilsitzelementes 17 umschlossen, wobei die Dichtfläche die Form eines Kreisringes aufweist, und im Zentrum des Kreises der Austritt des das Ventilsitzelement 17 durchsetzenden Flüssigkeitskanal 18 ist. Die Grundplatte 20 des Ventilsitzelementes 17 ist mit einem auf die Dichtfläche 22 zugerichteten Dichtansatz 23 versehen, der eine parallel zur Dichtfläche 22 angeordnete, komplementäre Dichtfläche 24 aufweist. In der Ausgangsstellung, somit nicht gedehnten Position der elastischen Membran 13, wie es in Figur 4 veranschaulicht ist, sind die Dichtflächen 22 und 24 von Ventilsitzelement 17 und Ventilkörper 15 in einem geringem Abstand voneinander angeordnet, der es gestattet, dass Flüssigkeit, vom Eintrittskanal 5 kommend, durch den Flüssigkeitskanal 18 über den zwischen den Dichtflächen 22 und 24 gebildeten Spalt in die Kammer 6 eintreten kann.

Der Ventilkörper 15 weist ferner mehrere, beispielsweise drei parallel zueinander angeordnete Distanzansätze 25 auf, die sich von der Grundplatte weg erstrecken und im Bereich ihrer freien Enden mit der elastischen Membran 13 verbunden sind.

Aufgrund der angegebenen Durchmesser von Eintrittskanal 5, Flüssigkeitskanal 18, Austrittskanal 7 und Flüssigkeitskanal 10 des Durchflussbegrenzers 8 ist der Widerstand, den der Austrittskanal 7 und der Flüssigkeitskanal 10 des Durchflussbegrenzers 8 dem Ausströmen der Flüssigkeit aus dem Gehäuse 2 entgegensetzen größer als der Widerstand, dem der Eintrittskanal 5 und der Flüssigkeitskanal 18 im Ventilsitzelement 17 dem Einströmen der Flüssigkeit in das Gehäuse 2 entgegensetzt. Bedingt durch das Einströmen der Flüssigkeit in das Gehäuse 2, verbunden mit dem Strömungswiderstand, hervorgerufen durch den Durchflussbegrenzer 8, entsteht in der mit Flüssigkeit gefüllten Kammer 6 ein hydrostatischer Druck, der auf den Ventilkörper 2 und die elastische Membran 13 wirkt. Bei drucklosem Anliegen der Distanzansätze 21 des Ventilkörpers 15 am Ventilsitzelement 17 wird der das Ventilsitzelement 17 durchsetzende Flüssigkeitskanal 18 nicht verschlossen und es kann durch den Spalt zwischen den Dichtflächen 22 und 24 Flüssigkeit aus dem Eintrittskanal 5 in die Kammer 6 und von dort in den Austrittskanal 7 strömen. Bedingt durch die Elastizität des Ventilsitzelementes 17 wird durch eine in Richtung auf diesen wirkende Kraft, bei Druckerhöhung der in der Kammer 6 befindlichen Flüssigkeit, die elastische Membran 13 geringfügig vom Kammerinneren weggewölbt und der Ventilkörper 6 in dieser Richtung bewegt, wobei die Distanzansätze 21 des Ventilkörpers 15 in das elastische Ventilsitzelement 17 eingedrückt werden. Hierdurch reduziert sich der Spalt zwischen den Dichtflächen 22 und 24. Der durch das Ventil 1 durchtretende Volumenstrom wird damit geringer. Bei ausreichend großer Kraft schließt der Ventilkörper 15 den Spalt, so dass im Eintrittskanal 5 befindliche Flüssigkeiten nicht mehr in die Kammer 6 eindringen kann. Diese zum Verschließen des Flüssigkeitskanals 18 bzw. zum Verringern des Spaltes erforderliche Kraft wird dadurch auf die Grundplatte 20 gelenkt, so dass sich beim Einströmen der Flüssigkeit in das Gehäuse 6 in diesem ein Druck aufbaut, der zur Auslenkung der elastischen Membran 13 derart führt, dass der mit dieser fest verbundene Ventilkörper 15 in Richtung des Ventilsitzelementes 17 gezogen wird.

Bedingt durch die durch den Austrittskanal 7 bzw. den Durchflussbegrenzer 8 austretenden Flüssigkeitsstrom baut sich in der Kammer 6 der Druck ab. Bei Druckabbau bedingt die Rückstellkraft der ausgelenkten elastischen Membran 13, dass der Ventilkörper 15 vom Ventilsitzelement 17 abgehoben wird, womit sich wieder der Spalt zwischen den Dichtflächen 22 und 24 bildet. Durch diese Rückkopplung zwischen dem in der Kammer 6 herrschenden Flüssigkeitsdruck und der in dem Ventilkörper 15 eingeleiteten Schließkraft stellt sich in der Kammer ein Gleichgewichtsdruck ein, der nicht von dem am Einlass des Eintrittskanals 5 anliegenden Druck abhängt, solange der dortige Druck größer ist als der sich bei ausreichend hohem Druck einstellende Gleichgewichtsdruck in der Kammer 6.

Üblicherweise benötigen hydraulische Ventile eine Abdichtung zwischen Regelelement und Gehäuse. Diese sind sehr reibungsbehaftet und eine starke Hysterese ist die Folge. Bei der Erfindung wird durch die schwimmende, berührungsfreie Aufhängung aller Funktionselemente diese Hysterese und damit ein schwingendes Regelverhalten ausgeschlossen.

## Patentansprüche

1. Ventil (1), insbesondere für medizinische und ernährungsphysiologische Flüssigkeiten, sowie Flüssigkeiten im biologischen und Laborbereich, mit einem Gehäuse (2), das eine Kammer (6), einen in Strömungsverbindung mit der Kammer (6) stehenden Eintrittskanal (5) und einen in Strömungsverbindung mit der Kammer (6) stehenden Austrittskanal (7) für die Flüssigkeit aufweist, wobei innerhalb der Kammer (6) ein Ventilkörper (15) zum Regeln des Durchflusses der Flüssigkeit durch die Kammer (6) angeordnet ist, **dadurch gekennzeichnet, dass** der Ventilkörper (15) beweglich ist, sowie mit einem elastischen Element (13) verbunden ist, das Bestandteil der Kammerwand (16) bildet.

2. Ventil nach Anspruch 1, **dadurch gekennzeichnet, dass** das Gehäuse (2) ein Ventilsitzelement (17) aufnimmt, dass ein Kanal (18) durchsetzt, der in Strömungsverbindung mit dem Eintrittskanal (5) steht.

3. Ventil nach Anspruch 2, **dadurch gekennzeichnet, dass** das Ventilsitzelement (17) als elastischer Körper ausgebildet ist.

4. Ventil nach einem der Ansprüche 1 bis 3, **dadurch gekennzeichnet, dass** der Ventilkörper (5) einen Regelabschnitt (22) aufweist, der in veränderlichem Abstand zur Austrittsöffnung des das Ventilsitzelement (17) durchsetzenden Kanals (18) oder zur Austrittsöffnung des Eintrittskanals (5) angeordnet ist.

5. Ventil nach Anspruch 4, **dadurch gekennzeichnet, dass** der Regelabschnitt (22) als parallel zum Gegenabschnitt (24) des Ventilsitzelementes (17) oder Gehäuses (2) angeordneter Oberflächenbereich des Ventilkörpers (15) ausgebildet ist.

6. Ventil nach einem der Ansprüche 3 bis 5, **dadurch gekennzeichnet, dass** der Ventilkörper (15) mindestens einen sich entweder am elastischen Ventilsitzelement (17) oder an der Innenwand des Gehäuses (6) elastisch abstützenden Distanzansatz (21) aufweist.

7. Ventil nach einem der Ansprüche 1 bis 6, **dadurch gekennzeichnet, dass** der Ventilkörper (15) im Gehäuse (2) verschieblich gelagert ist.

8. Ventil nach einem der Ansprüche 1 bis 7, **dadurch gekennzeichnet, dass** der Ventilkörper (15) fest mit dem elastischen Element (13) verbunden ist.

9. Ventil nach einem der Ansprüche 1 bis 8, **dadurch gekennzeichnet, dass** das elastische Element (13) als Membran ausgebildet ist.

10. Ventil nach Anspruch 9, **dadurch gekennzeichnet, dass** die elastische Membran (13) im Bereich ihres Randes im Gehäuse (2) abgedichtet gehalten ist.

11. Ventil nach Anspruch 10, **dadurch gekennzeichnet, dass** das Gehäuse (2) im Bereich der elastischen Membran (13) mit einem Schutzdeckel (12) versehen ist, wobei die Deckfläche des Deckels (12) in Abstand zur Membran (13) angeordnet ist.

12. Ventil nach einem der Ansprüche 1 bis 11, **gekennzeichnet durch** einen Durchflussbegrenzer (8), zum Begrenzen des **durch** den Austrittskanal (7) tretenden Volumenstromes.

13. Ventil nach Anspruch 12, **dadurch gekennzeichnet, dass** der Durchflussbegrenzer (8) in das Gehäuse (2) integriert oder in einen Anschluss (4) eines Gehäuses (2) eingesetzt ist.

14. Ventil nach Anspruch 12 oder 13, **dadurch gekennzeichnet, dass** der Durchflussbegrenzer (8) einen Flüssigkeitskanal (10) aufweist, dessen Durchflussquerschnitt geringer ist als der Durchflussquerschnitt des Austrittskanals (7).

15. Ventil nach einem der Ansprüche 1 bis 14, **dadurch gekennzeichnet, dass** der Durchflussquerschnitt des Austrittskanals (7) kleiner ist als der Durchflussquerschnitt des Eintrittskanals (5) und/oder der des Kanals (18) des Ventilsitzelementes (17).
